# EUROPÄISCHE PATENTSCHRIFT

(11) **EP 3 938 007 B1**
(45) Veröffentlichungstag und Bekanntmachung des Hinweises auf die Patenterteilung: **21.05.2025**
(21) Anmeldenummer: 20711845.6
(22) Anmeldetag: 11.03.2020
(51) Int. Cl.: A61M 1/16, A61M 1/32

(54) **KÜHLEINHEIT FÜR EINEN WÄRMETAUSCHER**
COOLING UNIT FOR A HEAT EXCHANGER
UNITÉ DE REFROIDISSEMENT POUR UN ÉCHANGEUR DE CHALEUR

(30) Priorität: 11.03.2019 DE 102019203253
(43) Veröffentlichungstag der Anmeldung: 19.01.2022
(73) Patentinhaber: ResuSciTec GmbH, 79108 Freiburg (DE)
(72) Erfinder: GRUDKE, Jürgen, 79108 Freiburg (DE); BENK, Christoph, 79108 Freiburg (DE)
(74) Vertreter: Rösler, Uwe
(86) Internationale Anmeldenummer: PCT/EP2020/056455
(87) Internationale Veröffentlichungsnummer: WO 2020/182856

(56) Entgegenhaltungen:
- EP-B1- 3 079 737
- DE-A1- 102017 211 671

## Beschreibung

### Technisches Gebiet

Die Erfindung bezieht sich auf eine Kühleinheit für einen in einem Oxygenator integrierten Wärmetauscher zur Temperierung von in einem extrakorporalen Blutkreislauf geführten Blut, mit einem eine Flüssigkeit bevorratenden Vorratsbehälter, einem ein Reaktionsmittel umfassenden Reaktionsbehälter, das in Verbindung mit der Flüssigkeit eine endotherme Reaktion zu initiieren vermag, einem einen fluidischen Zugang zwischen dem Vorratsbehälter und dem Reaktionsbehälter erzeugenden Funktionsmittel sowie einer zumindest bereichsweise innerhalb des Reaktionsbehälters verlaufenden Fluidleitung, die über eine Zu- und Ableitung verfügt, die jeweils fluiddicht mit einem Schlauchleitungssystem des Wärmetauschers verbindbar oder verbunden sind, und mit dem Schlauchleitungssystem des Wärmetauschers zumindest einen Teil eines Fluidkreislaufes bildet.

### Stand der Technik

Es ist bekannt die bei exothermen oder endothermen chemischen Reaktionen freigesetzte Wärme oder Kälte durch Wärmekopplung technisch zu nutzen. Für den Fall endothermer Reaktionen, bei denen Kälte freigesetzt wird, findet sich eine Vielzahl von Anwendungsfällen, die im Weiteren kurz skizziert werden.

Im Rahmen der Herzchirurgie oder Intensivmedizin insbesondere zur Behandlung von akutem Herz und/oder Lungenversagen finden Herz-Lungen-Maschinen Anwendung , mit denen die Pumpfunktion des Herzens sowie die Lungenfunktion für einen beschränkten Zeitraum ersetzbar sind. Das Blut verlässt dabei über einen extrakorporalen Blutkreislauf in Form eines Schlauchsystems den Körper, wird mit Hilfe eines Oxygenators, der Teil der Herz-Lungenmaschine ist, mit Sauerstoff angereichert und wieder in den Körper zurückgeführt. Der Oxygenator übernimmt dabei die Funktion der Lungen und führt dem Blut nicht nur den lebenswichtigen Sauerstoff zu, sondern entfernt gleichzeitig das durch Stoffwechselprozesse entstehende Kohlendioxid (CO2).

Alle heute verwendeten Oxygenatoren besitzen zusätzlich einen Wärmetauscher, mit dessen Hilfe sich das durchströmende Blut erwärmen aber insbesondere abkühlen lässt. So finden die Eingriffe am Herzen in der Regel unter Hypothermiebedingungen statt, d.h. das Blut wird mehr oder weniger stark abgekühlt, denn das Absenken der Körpertemperatur mindert die Stoffwechselaktivität der Zellen und erhöht die Ischämietoleranz der betroffenen Gewebe und Organe.

Als Kältequellen dienen sogenannte Hypothermiegeräte, in denen als Kühlflüssigkeit in der Regel abgekühltes Wasser erzeugt und genutzt wird, um das durch den Oxygenator strömende Blut über einen Wärmetauscher zu kühlen, d.h. dessen thermische Energie gezielt zu entziehen. Handelsübliche Hypothermiegeräte sind großbauende und schwergewichtige, zumeist auf Rollen gelagerte Bedieneinheiten, deren Heiz- und Kühlaggregate über Netzstrom versorgt werden und somit für einen Feldeinsatz nicht geeignet sind.

Moderne Hypothermiegeräte ermöglichen aufgrund einer kompakten Bauweise sowie einer weitgehend autonomen Strom- und Kühlwasserversorgung eine portable Nutzung, unabhängig von einer elektrischen Strom- und Wasserversorgung. Derartige Hypothermiegeräte sehen fluiddichte Anschlüsse für einen im Oxygenator integrierten Wärmetauscher vor, so dass sie modular oder integrativ i.V.m. einem Oxygenator eingesetzt werden können.

Das Prinzip der Kälteerzeugung beruht bei derartigen weitgehend autonom arbeitenden, modernen Hypothermiegeräten auf einer endothermen chemischen Reaktion zumeist zwischen Ammoniumnitrat, Kalzium-Ammonium-Nitrat oder Harnstoff und Wasser. Häufig findet Harnstoff als chemische Komponente Verwendung in Kältepacks, um einen raschen Kühleffekt zu generieren. Diese bestehen i.d.R. aus zwei abgetrennten Bereichen, von denen sich in einem Harnstoff befindet, im anderen Wasser. Wird die Trennung aufgehoben, löst sich der Harnstoff im Wasser. Da die Gitterenergie von Harnstoff größer ist als die Hydrationsenergie, entzieht der Lösungsvorgang der Umgebung Energie und kühlt diese ab, siehe Robert T. Sataloff: Sataloffs Comprehensive Textbook of Otolaryngology. Jaypee Brothers, 2016, ISBN 978-93-5152745-9, S. 412..

Die Druckschrift US 2014/0371552 A1 offenbart ein Blutzucker-Messgerät, das nicht invasiv auf der Hautoberfläche eines Patienten aufsetzbar ist und eine Kühlvorrichtung, eine Temperaturmessvorrichtung sowie eine Infrarotstrahlungsdetektor umfasst. Zum Zwecke der Blutzuckermessung wird die Hautoberfläche mittels der Kühlvorrichtung auf eine vorgegebene Temperatur gesenkt und die durch die Hautoberfläche emittierte oder absorbierte Infrarotstrahlung gemessen. Die Kühlvorrichtung besteht aus zwei fluiddicht abgeschlossenen und unmittelbar aneinander grenzenden Kammern, von denen eine Kammer mit Harnstoff und die andere mit Wasser befüllt ist. Mit Hilfe eines federkraftbeaufschlagten Dorns kann eine beide Kammern fluiddicht trennende Trennwand lokal perforiert werden, so dass sich das Wasser in die Kammer des Harnstoffes ergießt, wodurch eine Wärmeenergie aus der Umgebung entziehende endotherme Reaktion initiiert wird, die die zu vermessende Hautoberfläche vorgebbar abkühlt.

Eine Vorrichtung zum Temperieren auf Basis einer chemischen Reaktion sowie deren Verwendung als Temperiereinheit für einen Wärmetauscher ist in der Druckschrift DE 10 2017 211 671 A1 beschrieben. Die bekannte Vorrichtung nutzt einen mit granularen Harnstoff teilbefüllten Behälter, in dem ein mit Wasser befüllter zweiter Behälter mit einer verformbaren Behälterwand eingebracht ist. Zu Kühlzwecken ergießt sich das Wasser aus dem zweiten Behälter in den ersten Behälter und reagiert mit dem Harnstoff unter Ausbildung einer positiven Reaktionsenthalpie, die durch Wärmekopplung technisch nutzbar ist.

Die Druckschrift EP 3 079 737 B1 offenbart eine Kühleinheit für einen in einem Oxygenator integrierten Wärmetauscher, bei dem als Kältequelle eine chemische Reaktion von Zeolit als Sorptionsmittel und Wasser dient.

### Darstellung der Erfindung

Der Erfindung liegt die Aufgabe zugrunde, eine Kühleinheit für einen in einem Oxygenator integrierten Wärmetauscher zur Temperierung von in einem extrakorporalen Blutkreislauf geführten Blut, mit einem eine Flüssigkeit bevorratenden Vorratsbehälter, einem ein Reaktionsmittel umfassenden Reaktionsbehälter, das in Verbindung mit der Flüssigkeit eine endotherme Reaktion zu initiieren vermag, einem einen fluidischen Zugang zwischen dem Vorratsbehälter und dem Reaktionsbehälter erzeugenden Funktionsmittel sowie einer zumindest bereichsweise innerhalb des Reaktionsbehälters verlaufenden Fluidleitung, die über eine Zu- und Ableitung verfügt, die jeweils fluiddicht mit einem Schlauchleitungssystem des Wärmetauschers verbindbar oder verbunden sind, und mit dem Schlauchleitungssystem des Wärmetauschers zumindest einen Teil eines Fluidkreislaufes bildet, möglichst kompakt leichtgewichtig und autonom arbeitend auszubilden, um einen manuell portablen Einsatz zu ermöglichen. Die Kühleinheit soll mit möglichst einfachen und kostengünstigen Mitteln realisierbar sein und die Möglichkeit schaffen zumindest modular ausgebildete Teilkomponenten als Einwegprodukt bzw. als ein sogenanntes Disposabel auszubilden. Die von der Vorrichtung abgebbare Wärme- bzw. Kältemenge soll in möglichst kurzer Zeit bereitgestellt werden, um so eine möglichst große Kühl- bzw. Heizleistung zu erzeugen.

Die Lösung der der Erfindung zugrunde liegenden Aufgabe ist im Anspruch 1 angegeben. Die lösungsgemäße Kühleinheit in vorteilhafter Weise ausbildende Merkmale sind Gegenstand der Unteransprüche sowie der weiteren Beschreibung insbesondere unter Bezugnahme auf das illustrierte Ausführungsbeispiel zu entnehmen.

Die lösungsgemäße Kühleinheit nach den Merkmalen des Oberbegriffes des Anspruches 1 zeichnet sich dadurch aus, dass ein Ausgleichsbehälter mit einem Überlauf vorgesehen ist, in den die Flüssigkeit aus dem Vorratsbehälter nach erzeugtem fluidischen Zugang zwischen Vorratsbehälter und Reaktionsbehälter strömt. Der fluidische Zugang wird mittels eines Funktionsmittels hergestellt, das den Vorratsbehälter lokal zu perforieren bzw. zu öffnen vermag. Der Überlauf stellt eine Fluidverbindung zum Reaktionsbehälter dar, durch die ein Anteil, vorzugsweise der Hauptanteil, der bevorrateten Flüssigkeit in den Reaktionsbehälter zur Initiierung der endothermen Reaktion mit dem Reaktionsmittel, vorzugsweise in Form von granularem Harnstoff, gelangt. Die Fluidleitung ist fluidisch mit dem Ausgleichsbehälter verbunden, in den der Restanteil der bevorrateten Flüssigkeit aus dem Vorratsbehälter gelangt. Unter dem Restanteil der bevorrateten Flüssigkeit ist jener Flüssigkeitsanteil zu verstehen, der durch die Anordnungsgeometrie des Überlaufes relativ zum Fassungsvermögen des Ausgleichsbehälters nicht in den Reaktionsbehälter abfließt. Um zu verhindern, dass die durch den Überlauf in den Reaktionsbehälter gelangte Flüssigkeit zurück in den Ausgleichsbehälter fließen kann, ist längs der Fluidverbindung des Überlaufes vorzugsweise ein Rückschlag- bzw. Rückflußventil angeordnet. Zudem ist längs der Fluidleitung, die zumindest bereichsweise innerhalb des Reaktionsbehälters verläuft, eine außerhalb des Reaktionsbehälters befindliche Fluidpumpe angeordnet, vermittels der Flüssigkeit aus dem Ausgleichsbehälter in die zumindest bereichsweise innerhalb des Reaktionsbehälters verlaufende Fluidleitung gesaugt wird.

Die zumindest bereichsweise innerhalb des Reaktionsbehälters verlaufende Fluidleitung verläuft im Weiteren fluiddicht durch die den Reaktionsbehälter begrenzende Reaktionsbehälterwand nach außen und bildet einen Fluidleitungsabschnitt, der im Weiteren als Ableitung bezeichnet wird und in Form einer flexiblen Schlauchleitung ausgebildet ist. Die im Inneren verlaufende Fluidleitung ist aus Gründen eines möglichst optimierten Wärmeübergangs zum umgebenden Medium innerhalb des Reaktionsbehälters zumindest abschnittweise aus Metall, vorzugsweise aus Aluminium gefertigt. Um für eine möglichst großflächige Wärmeübergangskontaktfläche zwischen der Fluidleitung und der im Inneren des Reaktionsbehälters vorhandenen Flüssigkeit zu sorgen, ist der Leitungsweg der Fluidleitung im Inneren des Reaktionsbehälters möglichst lang gewählt. Hierzu ist die im Inneren des Reaktionsbehälters verlaufende Fluidleitung zumindest abschnittsweise vorzugsweise helikal oder wendelförmig ausgebildet.

Längs der nach Außen aus dem Reaktionsbehälter führenden Ableitung ist eine Fluidpumpe angeordnet und vorzugsweise in Form einer Rollenpumpe ausgebildet, durch die vermittels einer von außen auf die Schlauchleitung einwirkenden peristaltischen Druckausübung ein Fluidströmungsfluss derart in die Fluidleitung eingeprägt wird, so dass die Flüssigkeit, die sich innerhalb des Ausgleichsbehälters befindet, durch die Fluidleitung abgesaugt wird. Alternativ ist die Fluidpumpe stromauf zu dem vorstehenden helikal oder wendelförmig ausgebildeten Fluidleitungsabschnitt längs eines ausserhalb des Reaktionsbehälters geführten Fluidleitungsabschnitt angeordnet.

Optional ist längs der aus dem Reaktionsbehälter abführenden Ableitung eine Filtereinheit, vorzugsweise in Form eines Bakterienfilters angebracht, durch den eine Kontamination des Wärmetauschers im Oxygenator, durch Keime, bspw. in Form von Legionellen, vermieden wird.

Die Ableitung der Fluidleitung ist im Weiteren vorzugsweise über eine lösbar fluiddichte Kupplung mit dem Eingang eines Schlauchleitungssystems verbunden, das mit einem innerhalb eines Oxygenators integrierten Wärmetauschers thermisch gekoppelt ist.

Der fluidische Ausgang des thermisch an den Wärmetauscher koppelnden Schlauchleistungssystems ist vorzugsweise über eine lösbar fluiddichte Kupplung mit der Zuleitung der Fluidleitung verbunden, die in den Ausgleichsbehälter mündet. Somit bilden der Ausgleichsbehälter, die Fluidleitung sowie auch das Schlauchleitungssystem des Wärmetauschers einen in sich geschlossenen Fluidkreislauf, längs dem die Flüssigkeit zirkuliert, die als Wärmeträgerflüssigkeit des Wärmetauschers dient. Eben dieser als Wärmeträgerflüssigkeit dienende Flüssigkeitsanteil stammt aus der innerhalb des Vorratsbehälters bevorrateten Flüssigkeit, die sich nach entsprechender lokaler Perforation bzw. Öffnung des Vorratsbehälters mit Hilfe des im Weiteren noch näher zu erläuternden Funktionsmittels in den Ausgleichsbehälter ergießt.

Aufgrund eines nur begrenzten Aufnahmevolumens innerhalb des Ausgleichsbehälters, das kleiner als das Fassungsvermögen des Vorratsbehälters ist, fließt der größte Flüssigkeitsanteil über den Überlauf längs der Fluidverbindung in den Reaktionsbehälter, in dem das Reaktionsmittel bevorratet ist, vorzugsweise in Form von granularem Harnstoff. Ebenso sind auch alternative Reaktionsmittel geeignet, die mit einer Flüssigkeit, vorzugsweise Wasser, unter Ausbildung einer endothermen chemischen Reaktion aus der Umgebung thermische Energie entziehen.

Die innerhalb des Vorratsbehälters bevorratete Flüssigkeitsmenge ist derart bemessen, so dass der über den Überlauf und der Fluidverbindung in den Reaktionsbehälter abfließende Flüssigkeitsanteil wenigstens 70% beträgt, wohingegen der Restanteil der Flüssigkeit innerhalb des Ausgleichsbehälter zurückgehalten wird, der, wie vorstehend erläutert, als Wärmeträgerflüssigkeit für den fluidisch mit der lösungsgemäß ausgebildeten Kühleinheit verbundenen Wärmetauschers dient. Die Mengenaufteilung der Flüssigkeit, die vom bzw. aus dem Ausgleichsbehälter über die Fluidverbindung in den Reaktionsbehälter abfließt, ist insbesondere durch die Rohrleitungshöhe, mit der die Fluidverbindung in den Ausgleichsbehälter ragt, vorgebbar.

Um das Befüllen des Reaktionsbehälters bzw. den Abfließvorgang der Flüssigkeit aus dem Ausgleichsbehälter in den Reaktionsbehälter durch die Fluidverbindung hindurch, aufgrund einer sich ansonsten innerhalb des Reaktionsbehälters ausbildende Druckerhöhung nicht zu behindern, sieht der Reaktionsbehälter wenigstens eine Entlüftungsöffnung im oberen Bereich nahe des Ausgleichsbehälter in die Umgebung vor. Die Entlüftungsöffnung weist vorzugsweise einen hydrophoben Filtereinsatz auf, durch den ein unkontrollierter Flüssigkeitsaustritt, bedingt durch Kippen oder Bewegen der Kühleinheit, unterbunden werden kann.

Innerhalb des Reaktionsbehälters ist zudem zur Unterstützung und Homogenisierung der chemischen, endothermen Reaktion zwischen der Flüssigkeit und dem granularen Reaktionsmittel ein Rührwerk angeordnet, das über eine mechanische Schnittstelle, bspw. in Form eines Zahnradgetriebes, mit einem außerhalb des Reaktionsbehälters angeordneten Antriebsmotor antreibbar ist. Um sicherzustellen, dass keine Flüssigkeitsanteile aus dem Reaktionsbehälter in den Ausgleichsbehälter zurückgelangen können, zumal hierdurch die innerhalb des Ausgleichsbehälters befindliche Flüssigkeit kontaminiert würde, ist längs der Fluidverbindung ein Rückschlagventil angeordnet.

Zum Zwecke einer möglichst einfachen und fehlerfreien Handhabung der lösungsgemäß ausgebildeten Kühleinheit ist der Vorratsbehälter vorzugsweise in Form eines Flüssigkeitsbeutels ausgebildet. Der Flüssigkeitsbeutel befindet sich auch aus Gründen einen mechanischen Schutzes innerhalb einer ersten Umhausung, die vertikal über einer zumindest den Ausgleichsbehälter umgebenden zweiten Umhausung angeordnet ist. Vertikal unterhalb der den Ausgleichsbehälter umgebenden zweiten Umhausung ist eine dritte, den Reaktionsbehälter umfassende Umhausung angeordnet. Optional kann die zweite und dritte Umhausung einstückig ausgebildet sein. Zwischen der ersten und zweiten Umhausung ist zusätzlich ein Abstandshalter angeordnet, der einen vorgegebenen vertikalen Abstand zwischen dem in der ersten Umhausung enthaltenen Vorratsbehälter und dem vorzugsweise am oder innerhalb des Ausgleichsbehälters fest angebrachten Funktionsmittel sichert.

Das Funktionsmittel ist in Form eines scharfkantigen Objektes ausgebildet und vorzugsweise vertikal unterhalb des Vorratsbehälters angeordnet.

Der den Vorratsbehälter vertikal gegenüber des Ausgleichsbehälters vertikal beabstandenden Abstandshalter, der einen unmittelbaren Kontakt zwischen dem Vorratsbehälter und dem scharfkantigen Funktionsmittel verhindert, ist zwischen der ersten und zweiten Umhausung vorzugsweise derart gelagert, so dass der Abstandshalter seitlich aus dem Stapelverbund separierbar ist, beispielsweise durch manuelles Herausziehen. Nach Entfernen des Abstandshalters fällt der Vorratsbehälter aufgrund seiner Gewichtskraft und der Schwerkraft folgend vertikal nach unten und tritt in Kontakt mit dem scharfkantigen Funktionsmittel, wodurch der Vorratsbehälter mechanisch lokal perforiert bzw. geöffnet wird, so dass sich die im Vorratsbehälter bevorratete Flüssigkeit vollständig in den Ausgleichsbehälter ergießt. Zeitgleich mit dem vertikalen Absenken bzw. Herabfallen des Vorratsbehälters und der damit verbundenen Befüllung des Ausgleichs- und Reaktionsbehälters werden die Fluidpumpe sowie auch der Antriebsmotor zur Aktivierung des Rührwerkes innerhalb des Reaktionsbehälters in Betrieb genommen.

Die den Vorratsbehälter umgebende erste Umhausung sowie die vertikal darunter angeordnete zweite Umhausung sind derart in Bezug auf ihre vertikal unmittelbar einander zugewandten Seiten derart ausgebildet, dass nach Entfernen des Abstandshalters beide Umhausungen mechanisch eindeutig, bspw. in Art einer umlaufenden Feder-Nut-Verbindung, unter Ausbildung einer festen mechanischen Fügeverbindung ineinander gleiten.

Die lösungsgemäße Kühleinheit dient zur schnellen und effiziente Bereitstellung einer großen Kühlleistung innerhalb kürzester Zeit, die außer der Inbetriebnahme der Fluidpumpe sowie auch des Antriebsmotors keine darüber hinaus erforderliche elektrische Stromversorgung bedarf. Aufgrund des nur begrenzten Energiebedarfes kann die erforderliche elektrische Energie im Rahmen einer Batterie zur Verfügung gestellt werden.

Eine bevorzugte Ausgestaltung der lösungsgemäßen Kühleinheit sieht einen modularen Aufbau derart vor, so dass die elektrischen Komponenten, wie Fluidpumpe und Antriebsmotor nebst dazu erforderlicher Steuereinheit und elektrischen Energiequelle, in einem modular ausgebildeten, einheitlichen Gehäuse untergebracht sind. Die den Vorratsbehälter umfassende erste Umhausung, der Abstandshalter, die den Ausgleichsbehälter umfassende zweite Umhausung sowie die den Reaktionsbehälter umfassende dritte Umhausung sind jeweils als vertikal übereinander stapelbare Moduleinheiten ausgebildet und können in Form von Einwegartikeln nach Gebrauch entsorgt werden. Vorzugsweise bietet es sich an zumindest den Vorratsbehälter und den Reaktionsbehälter aus einem kunststoffbasierten Leichtverpackungsmaterial zu fertigen, bspw. dicht gepresstes Polystyrol, das einer Werkstoffwiederverwertung zugeführt werden kann. Um sicherzustellen, dass der Reaktionsbehälter flüssigkeitsdicht ist, ist die mit der Flüssigkeit in Kontakt tretende Innenwand des Reaktionsbehälters mit einer flüssigkeitsdichten Beschichtung versehen. Zudem gilt es das Beschichtungsmaterial so zu wählen, dass es chemisch inert gegenüber des sich ausbildenden Flüssigkeits-Reaktionsmittel-Gemisches und der sich daraus ausbildenden chemischen Produkte ist.

Aus Gründen von Nachhaltigkeit und einer schonenden Entsorgung ist es zudem vorteilhaft innerhalb des Reaktionsbehälters ein Bindemittel vorzusehen, das in Reaktion mit der Flüssigkeit und/oder dem sich ausbildenden Flüssigkeits-/Reaktionsmittel-Gemisch einen gelbildenden Prozess auslöst, so dass nach Gebrauch der Kühleinheit der Reaktionsbehälter aufgrund der gelierten Masse im Inneren des Behälters als Hausmüll entsorgt werden kann und so keine kostenaufwendigen Entsorgung bedingt. Vorzugsweise sollte die chemische Reaktion mit dem Bindemittel zeitverzögert zur endothermen Reaktion zwischen der Flüssigkeit und dem Reaktionsmittel erfolgen, so dass für eine vollständige Reaktion zwischen der Flüssigkeit und dem Reaktionsmittel gesorgt ist. Hierzu bietet es sich an das Bindemittel mit einem flüssigkeitslöslichen Material zu kapseln und im Inneren des Reaktionsbehälters zu bevorraten oder über einen am Reaktionsbehälter angebrachten Zugabemechanismus zeitverzögert in das Innere des Reaktionsbehälters zu verabreichen. Im Falle von Wasser als Flüssigkeit eignet sich vorzugsweise Xanthan als Bindemittel.

Die Kühleinheit kann grundsätzlich mit einem Wärmetauscher für beliebige Anwendungen fluidisch verbunden und eingesetzt werden. Denkbar sind Wärmetauscher in Form von Kühlflächen oder Kühlmatten, ebenso wie Kühlgefäße oder Kühlfässer, um nur einige Anwendungen zu nennen. Die Kühleinheit eignet sich als Kältequelle für Wärmetauscher, die in stationären oder portablen Kühlaggregaten integriert sind.

### Kurze Beschreibung der Erfindung

Die Erfindung wird nachstehend ohne Beschränkung des allgemeinen Erfindungsgedankens anhand eines Ausführungsbeispiels unter Bezugnahme auf die Zeichnung exemplarisch beschrieben. Es zeigt:
- Fig. 1: schematisierte Darstellung einer lösungsgemäß ausgebildeten Kühleinheit zur Temperierung eines in einem Oxygenator integrierten Wärmetauschers im Stadium vor der Aktivierung der Kühlfunktion,
- Fig. 2: schematisierte Darstellung der Kühleinheit nach Aktivierung der Kühlfunktion und
- Fig. 3: Alternative Ausgestaltung der lösungsgemäßen Kühleinheit.

### Wege zur Ausführung der Erfindung, gewerbliche Verwendbarkeit

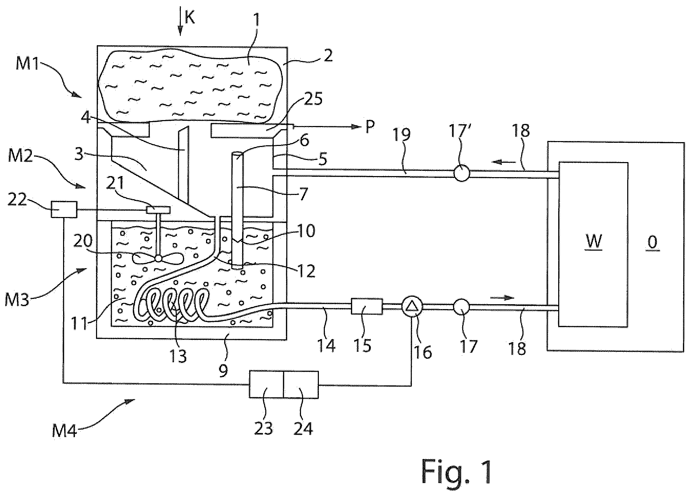

Figur 1 illustriert in schematischer Darstellung eine lösungsgemäß ausgebildete Kühleinheit K zur Bereitstellung einer gekühlten Wärmeträgerflüssigkeit zum Betrieb eines Wärmetauschers W, der vorzugsweise Teil eines Oxygenators O ist.

Die Kühleinheit K weist im Wesentlichen vier Module M1 bis M4 auf, von denen zumindest die Module M1-M3 nach dem Baukastenprinzip vertikal übereinander zusammensetzbar sind. Das Modul M1 weist einen Vorratsbehälter 1 für eine Flüssigkeit, vorzugsweise in Form von Wasser auf. Vorzugsweise besteht der Vorratsbehälter 1 aus einem mit Wasser befüllten Plastikbeutel oder Plastikkanister, der zum Schutz und für eine mechanische Fügung mit dem darunter befindlichen Modul M2 von einer ersten Umhausung 2 zumindest teilweise umgeben ist.

Vertikal unterhalb des ersten Moduls M1 ist das zweite Modul M2 angeordnet, das einen Ausgleichsbehälter 3 umfasst, in dem ein Funktionsmittel 4 in Form eines vertikal nach oben scharfkantig zulaufenden Objektes, bspw. Nadel, Dorn etc., fest angeordnet ist. Der Ausgleichsbehälter 3 ist von einer zweiten Umhausung 5 umgeben. In das Innere des Ausgleichsbehälters 3 ragt vertikal von unten ein Überlauf 6 mit einer Fluidverbindung 7, die vertikal von oben in den Reaktionsbehälter 8 des dritten Moduls M3 mündet, der von einer dritten Umhausung 9 umgeben ist. Die Fluidverbindung 7 ist als beidseitig offene Rohrleitung ausgebildet und weist ein Rückschlagventil 10 auf, das einen Flüssigkeitseintrag von Seiten des Reaktionsbehälters 8 in den Ausgleichsbehälter 3 verhindert. In dem Reaktionsbehälter 8 ist ein Reaktionsmittel 11 in granularer Form bevorratet, das vorzugsweise aus granularem Harnstoff besteht.

Ferner mündet im Bodenbereich des Ausgleichsbehälters 3 eine Fluidleitung 12, die innerhalb des Reaktionsbehälters 11 weiter verläuft, vorzugsweise unter Ausbildung von Leitungswendeln 13, um eine möglichst große Fluidleitungsoberfläche innerhalb des Reaktionsbehälters 8 zu realisieren. Die Fluidleitung 12 führt fluiddicht durch die dritte Umhausung 9 nach außen und dient im Weiteren als Ableitung 14 der Kühleinheit K. Die im Inneren des Reaktionsbehälters 9 verlaufende Fluidleitung 12 und insbesondere die dort befindlichen Leitungswendeln 13 sind aus einem Wärme sehr gut leitenden Material gefertigt, vorzugsweise aus Metall, wohingegen die Fluidleitung längs der Ableitung 14 aus einem thermisch schlecht leitenden und elastischen Material, bspw. Kunststoff, besteht.

Längs der Ableitung 14 ist eine Filtereinheit 15, vorzugsweise in Form eines Bakterienfilters, eingesetzt. Stromab zur Filtereinheit 15 ist längs der Ableitung 14 eine Fluidpumpe 16, vorzugsweise in Form einer Rollenpumpe, angeordnet. Stromab der Fluidpumpe 16 ist eine lösbare fluiddichte Kupplung 17 für eine fluiddichte Verbindung mit einem dem Wärmetauscher W zugehörigen Schlauchleitungssystem 18.

In gleicher Weise ist das Schlauchleitungssystem 18 mit einer lösbar fluiddichten Kupplung 17' mit der als Zuleitung 19 in den Ausgleichsbehälter 3 der Kühleinheit K dienenden Fluidleitung verbunden.

Das dritte Modul M3 verfügt überdies über ein Rührwerkzeug 20, das über eine lösbare Getriebeeinheit 21 mit einem Antriebsmotor 22 gekoppelt ist. Der Antriebsmotor 22, die Fluidpumpe 16 sowie eine elektronische Steuereinheit 23 in Kombination mit einer elektrischen Energiequelle 24, die zum Betrieb des Antriebsmotors 22 und der Fluidpumpe 15 dienen, bilden das vierte Modul M4, das von einer nicht weiter dargestellten vierten Umhausung umgeben ist.

Das erste und zweite Modul M1, M2 sind vertikal durch einen Abstandshalter 25 vertikal beabstandet, so dass das Funktionsmittel 4 in Form eines vertikal nach oben spitz zulaufenden Objektes den innerhalb des ersten Moduls M1 enthaltenen Vorratsbehälter 1 nicht berührt. Der Abstandshalter 25 ist gleitend zwischen dem ersten und zweiten Modul M1, M2 gelagert und vorzugsweise mit Hilfe eines nicht dargestellten Rastmechanismus gegenüber eines unkontrollierten seitlichen Herausrutschens gesichert.

Zur Aktivierung der Kühleinheit K gilt es den Abstandshalter 25 seitlich aus dem vertikalen Modulverbund M1, M2 zu entfernen, beispielsweise durch seitliches manuelles Herausziehen, siehe Pfeildarstellung P.

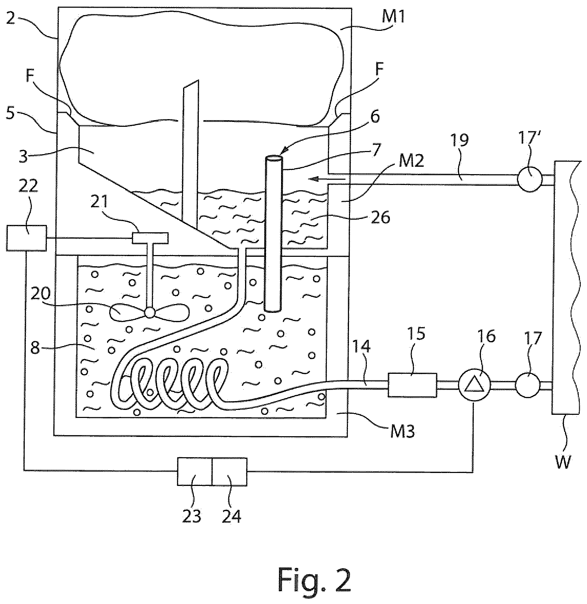

Figur 2 illustriert den Zustand, nach einem seitlichen Entfernen des Abstandshalters 25 aus dem modularen Stapelverbund. Als Folge des fehlenden Abstandshalters 25 fällt der Vorratsbehälter 1 samt erster Umhausung 2 vertikal nach unten, wodurch das nach oben spitz zulaufende Funktionsmittel 4 den Vorratsbehälter 1 lokal perforiert. Um sicher zu stellen, dass der Fall- und Fügevorgang des ersten Moduls M1 auf und in das zweite Modul M2 in einer bestimmten Weise erfolgt, weisen die erste und zweite Umhausungen 2, 5 an ihren jeweils vertikal zugewandten Seiten seitlich umlaufende Fügekonturen F auf.

Infolge der mechanisch initiierten Perforation des Vorratsbehälters 1 strömt der gesamte Flüssigkeitsinhalt des Vorratsbehälters 1 in den Ausgleichsbehälter 3. Etwa 80% der im Vorratsbehälter 1 bevorrateten Flüssigkeitsmenge strömt über den Überlauf 6 und die Fluidverbindung 7 in den Reaktionsbehälter 8 und initiiert mit dem Reaktionsmittel 11 eine endotherme chemische Reaktion, wodurch innerhalb des Reaktionsbehälters 8 eine Abkühlung erfolgt. Ein Restanteil 26 der Flüssigkeit bleibt innerhalb des Ausgleichsbehälters 3 und dient als Wärmeträgerflüssigkeit zum Betrieb des Wärmetauschers W. Zeitgleich mit der Entfernung des Abstandshalters 25 und der dadurch schwerkraftgetriebenen Perforation des Vorratsbehälters 1 werden vermittels der elektronischen Steuereinheit 23 sowohl die Fluidpumpe 16 als auch der Antriebsmotor 22, der das Rührwerkzeug 20 über die Getriebeeinheit 21 antreibt, aktiviert. Die als Saugpumpe arbeitende Fluidpumpe 16 saugt die als Restanteil 26 innerhalb des Ausgleichsbehälters 3 befindliche Flüssigkeit durch die Fluidleitung 12, die aufgrund der Abkühlung innerhalb des Reaktionsbereiches 8 abgekühlt wird. Um den Wärmeübergang von der innerhalb der Fluidleitung 12 geführten Flüssigkeit auf das sich im Wege der endothermen Reaktion abkühlende Flüssigkeits-/Reaktionsmittel-Gemisch zu optimieren gilt es für eine möglichst große Wärmeübergangskontaktfläche zwischen der Fluidleitung 12 und des im Inneren des Reaktionsbehälters 8 vorhandenen Flüssigkeits-/Reaktionsmittel-Gemisches zu sorgen. Hierzu ist der Leitungsweg der Fluidleitung 12 im Inneren des Reaktionsbehälters 8 zumindest abschnittsweise helikal oder wendelförmig ausgebildet.

Die abgekühlte Flüssigkeit wird über die Ableitung 14 durch die Filtereinheit 15 in den Wärmetauscher W gepumpt. Die nach Austritt aus dem Wärmetauscher W abströmende Wärmeträgerflüssigkeit gelangt über die Zuleitung 19 zurück in den Ausgleichsbehälter 3, aus dem Flüssigkeit erneut über die Fluidleitung 12 zum Zwecke ihrer Kühlung in den Bereich des Reaktionsbehälters 8 gesaugt wird.

In vorteilhafter Weise sind das erste, zweite und dritte Modul M1, M2, M3 als Einwegartikel ausgebildet, wohingegen das die elektrischen Komponenten umfassende vierte Modul M4 beliebig oft wiederverwendet werden kann. Die Filtereinheit 15 ist vorzugsweise integraler Bestandteil des dritten Moduls M3 und somit ebenfalls Teil eines Einwegartikels. Aus Gewichts- und Kostengründen sind die Module M1, M2 und M3 aus kunststoffbasierten Leichtverpackungsmaterial gefertigt, das zudem einer Werkstoffwiederverwertung zugeführt werden kann. Zudem sind der Ausgleichsbehälter 3 und der Reaktionsbehälter 8, d.h. das Modul 2 und 3 inwandig mit einer flüssigkeitsdichten Beschichtung versehen.

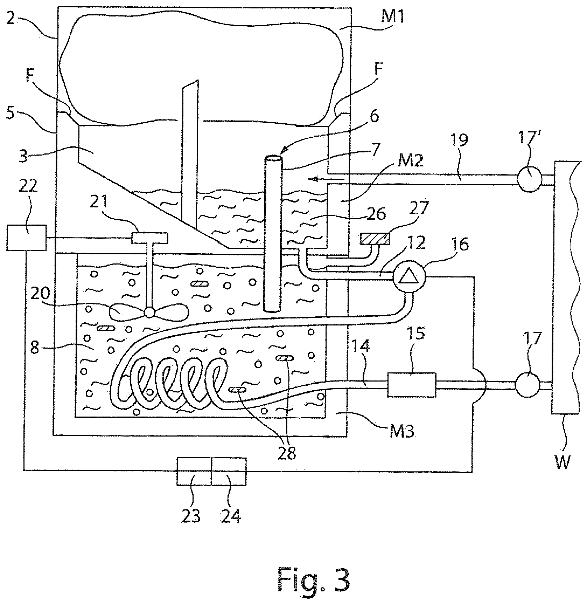

In Figur 3 ist eine weiter bevorzugte Ausführungsform für die Ausbildung der Kühleinheit im Zustand der unmittelbar vertikal aufsitzenden Module M1, M2 und M3, vergleichbar mit der Darstellung in Figur 2, illustriert. All jene Komponeten, die mit den bereits erläuterten Komponenten identisch sind, sind mit den bereits eingeführten Bezugszeichen versehen.

Im Unterschied zur Darstellung in Figur 2 führt die Fluidleitung 12 unmittelbar stromab zu ihrem fluidischen Anschluß an den Ausgleichsbehälter 3 nach Außen, d.h. außerhalb des Reaktionsbehälters 8, wo die Fluidpumpe 16 längs der Fluidleitung 12 eingebracht ist und Flüssigkeit aus dem Ausgleichsbehälter 3 in die Fluidleitung 12 saugt. Stromab der außerhalb des Reaktionsbehälters 8 angeordneten Fluidpumpe 16 führt die Fluidleitung 12 wieder zurück in den Reaktionsbehälter 8, innerhalb dem die Fluidleitung 12 helikal geformt ist, um eine möglichst große Wärmeübergangskontaktfläche zu bilden und für eine effektive Abkühlung der innerhalb der Fluidleitung 12 geführten Flüssigkeit zu sorgen.

Längs der nach außerhalb des Reaktionsbehälters 8 führenden Ableitung 14 ist die Filtereinheit 15 angeordnet, die als Bakterienfilter, bspw. in Form eines Legionellenfilters, für eine Keimfreiheit der gekühlten Flüssigkeit sorgt, um auf diese Weise den Wärmetauscher W innerhalb des Oxygenators nicht zu kontaminieren.

Zudem sieht der Reaktionsbehälter 8 im oberen Bereich eine Entlüftungseinheit 27 mit einem hydrophoben Filter vor, die für eine vollständige und schnelle Befüllung des Reaktionsbehälters 8 sorgt und ein unkontrolliertes Entweichen von Flüssigkeit nach Außen verhindert.

Nach Beendigung des Kühlvorganges sorgt ein im Reaktionsbehälter 8 bevorratetes Bindemittel 28, vorzugsweise Xanthan, für eine Gelierung des Flüssigkeits-Reaktionsmittel-Gemisches, so dass eine einfache Entsorgung der Module 1, 2 und 3, bspw. im Rahmen des Hausmülls möglich ist. Das Bindemittel 27 ist hierzu mit einem flüssigkeitslöslichen Material gekapselt, das sich nach einer gewissen Verweildauer innerhalb der Flüssigkeit vollständig auflöst und das Bindemittel innerhalb des Reaktionsbehälters freisetzt.

### Bezugszeichenliste

- 1: Vorratsbehälter
- 2: erste Umhausung
- 3: Ausgleichsbehälter
- 4: Funktionsmittel
- 5: zweite Umhausung
- 6: Überlauf
- 7: Fluidverbindung
- 8: Reaktionsbehälter
- 9: dritte Umhausung
- 10: Rückschlagventil
- 11: Reaktionsmittel
- 12: Fluidleitung
- 13: Leitungswendel
- 14: Ableitung
- 15: Filtereinheit
- 16: Fluidpumpe
- 17, 17': lösbar fluiddichte Kupplung
- 18: Schlauchleitungssystem
- 19: Zuleitung
- 20: Rührwerkzeug
- 21: Getriebeeinheit
- 22: Antriebsmotor
- 23: elektrische Steuereinheit
- 24: elektrische Energiequelle, Batterie
- 25: Abstandshalter
- 26: Restanteil der Flüssigkeit
- 27: Entlüftungseinheit
- 28: Bindemittel
- W: Wärmetauscher
- O: Oxygenator
- M1,M2,M3,M4: Module
- P: Pfeilrichtung
- K: Kühleinheit
- F: Fügekontur

## Patentansprüche

1. Kühleinheit für einen in einem Oxygenator (O) integrierten Wärmetauscher (W) zur Temperierung von in einem extrakorporalen Blutkreislauf geführten Blut, mit
- einem eine Flüssigkeit bevorratenden Vorratsbehälter (1),
- einem ein Reaktionsmittel (11) umfassenden Reaktionsbehälter (8), das in Verbindung mit der Flüssigkeit eine endotherme Reaktion zu initiieren vermag,
- einem einen fluidischen Zugang zwischen dem Vorratsbehälter (1) und dem Reaktionsbehälter (8) erzeugenden Funktionsmittel (4) sowie
- einer zumindest bereichsweise innerhalb des Reaktionsbehälters (8) verlaufenden Fluidleitung (12), die über eine Zu- und Ableitung (14, 19) verfügt, die jeweils fluiddicht mit einem Schlauchleitungssystem (18) des Wärmetauschers (W) verbindbar oder verbunden sind, und mit dem Schlauchleitungssystem (18) des Wärmetauschers (W) zumindest einen Teil eines Fluidkreislaufes bildet,
**dadurch gekennzeichnet, dass** ein Ausgleichsbehälter (3) mit einem Überlauf (6) vorgesehen ist, in den die Flüssigkeit aus dem Vorratsbehälter (1) nach erzeugtem fluidischen Zugang zwischen Vorratsbehälter (1) und Reaktionsbehälter (8) strömt, dass der Überlauf (6) eine Fluidverbindung (7) zum Reaktionsbehälter (8) darstellt, durch die ein Anteil der bevorrateten Flüssigkeit in den Reaktionsbehälter (8) zur Initiierung der endothermen Reaktion mit dem Reaktionsmittel (11) gelangt,
dass die Fluidleitung (12) fluidisch mit dem Ausgleichsbehälter (3) verbunden ist, in den ein Restanteil (26) der bevorrateten Flüssigkeit aus dem Vorratsbehälter (1) gelangt, und
dass längs der Fluidleitung (12) eine Fluidpumpe (16) angeordnet ist, vermittels der Flüssigkeit aus dem Ausgleichsbehälter (3) in die zumindest bereichsweise innerhalb des Reaktionsbehälters (8) verlaufende Fluidleitung (12) gelangt.

2. Kühleinheit nach Anspruch 1,
**dadurch gekennzeichnet, dass** längs der Fluidverbindung (7) des Überlaufes (6) ein Rückschlagventil (10) angeordnet ist, das ein Rückströmen der Flüssigkeit aus dem Reaktionsbehälter (8) in den Ausgleichsbehälter (3) verhindert.

3. Kühleinheit nach Anspruch 1 oder 2,
**dadurch gekennzeichnet, dass** die Fluidpumpe (16) in Form einer Saugeinheit ausgebildet ist, die längs der Fluidleitung (12) außerhalb des Ausgleich- und Reaktionsbehälters (3, 8) angeordnet ist.

4. Kühleinheit nach Anspruch 1 oder 2,
**dadurch gekennzeichnet, dass** die Fluidpumpe (16) als Rollenpumpe ausgebildet ist.

5. Kühleinheit nach einem der Ansprüche 1 bis 4,
**dadurch gekennzeichnet, dass** die Fluidleitung (12) stromab zu dem innerhalb des Reaktionsbehälters (8) verlaufenden Bereich die Ableitung (14) vorsieht, und
dass längs der außerhalb des Reaktionsbehälters verlaufenden Ableitung (14) eine Filtereinheit (15) angeordnet ist.

6. Kühleinheit nach Anspruch 5,
**dadurch gekennzeichnet, dass** die Filtereinheit (15) ein Bakterienfilter, insbesondere ein Legionellenfilter ist.

7. Kühleinheit nach einem der Ansprüche 1 bis 6,
**dadurch gekennzeichnet, dass** die Zuleitung (19) der Fluidleitung (12) in den Ausgleichsbehälter (3) mündet, und
dass der Fluidkreislauf aus dem Ausgleichsbehälter (3), der Fluidleitung (12) sowie dem Schlauchleitungssystem des Wärmetauschers (W) besteht, durch den der Restanteil (26) der Flüssigkeit als Wärmeträgerflüssigkeit des Wärmetauschers (W) zirkuliert.

8. Kühleinheit nach einem der Ansprüche 1 bis 7,
**dadurch gekennzeichnet, dass** der Vorratsbehälter (1) vertikal oberhalb des Ausgleichsbehälters (3) angeordnet ist,
dass das Funktionsmittel (4) in Form eines scharfkantigen Objektes ausgebildet ist, das vertikal unterhalb des Vorratsbehälters (1) angeordnet ist,
dass zwischen dem Vorratsbehälter (1) und dem Ausgleichsbehälter (3) ein Abstandshalter (25) angeordnet ist, der den Vorratsbehälter (1) vertikal über dem Funktionsmittel (4) auf Abstand hält, und
dass der Vorratsbehälter (1) nach Entfernen des Abstandshalters (25) Schwerkraftsgetrieben in Kontakt mit dem Funktionsmittel (4) tritt, das infolgedessen den Vorratsbehälter (1) mechanisch lokal durchdringt, sodass sich die im Vorratsbehälter (1) bevorratete Flüssigkeit vollständig in den Ausgleichsbehälter (3) ergießt.

9. Kühleinheit nach einem der Ansprüche 1 bis 8,
**dadurch gekennzeichnet, dass** das Funktionsmittel (4) am Ausgleichsbehälter (3) fest angeordnet ist.

10. Kühleinheit nach einem der Ansprüche 1 bis 9,
**dadurch gekennzeichnet, dass** innerhalb des Reaktionsbehälters (8) ein Rührwerk (20) angeordnet ist, das über eine mechanische Schnittstelle mit einem außerhalb des Reaktionsbehälters (8) angeordneten Antriebmotors (22) antreibbar ist.

11. Kühleinheit nach einem der Ansprüche 1 bis 10,
**dadurch gekennzeichnet, dass** zumindest der Vorratsbehälter (1), das Funktionsmittel (4) und der Reaktionsbehälter (8) als Einwegartikel ausgebildet sind.

12. Kühleinheit nach den Ansprüchen 5, 10 und 11,
**dadurch gekennzeichnet, dass** die Filtereinheit (15) und das Rührwerk (20) Teile des Einwegartikels sind und der Antriebsmotor (22) und die Fluidpumpe (16) mit einer elektrischen Energiequelle (24) sowie einer elektrischen Steuerung (23) verbunden und als modulare Einheit für eine mechanische Adaption an den Einwegartikel ausgebildet sind.

13. Kühleinheit nach einem der Ansprüche 1 bis 12,
**dadurch gekennzeichnet, dass** der Wärmetauscher (W) in einem stationären oder portablen Kühlaggregat integriert ist.

14. Kühleinheit nach einem der Ansprüche 1 bis 13,
**dadurch gekennzeichnet, dass** innerhalb des Reaktionsbehälters (8) ein Bindemittel bevorratet ist, das bei Kontakt mit der in den Reaktionsbehälter (8) eintretenden Flüssigkeit diese zu gelieren vermag.

15. Kühleinheit nach einem der Ansprüche 1 bis 14,
**dadurch gekennzeichnet, dass** zumindest der Vorratsbehälter (1) und der Reaktionsbehälter (8) aus einem kunststoffbasierten Leichtverpackungsmaterial gefertigt sind.

16. Kühleinheit nach Anspruch 15,
**dadurch gekennzeichnet, dass** zumindest die mit der Flüssigkeit in Kontakt tretende Innenwand des Reaktionsbehälters mit einer flüssigkeitsdichten Beschichtung versehen ist.

17. Kühleinheit nach einem der Ansprüche 1 bis 16,
**dadurch gekennzeichnet, dass** der Reaktionsbehälter (8) eine Entlüftungseinheit vorsieht.

## Claims

1. A cooling unit for a heat exchanger (W), integrated in an oxygenator (O), for the purpose of controlling the temperature of blood conveyed in an extracorporeal blood circuit,
comprising:
- a reservoir (1) in which a liquid is stored,
- a reaction vessel (8) comprising a reactant (11) which, in conjunction with the liquid, can initiate an endothermal reaction,
- functional means (4) for providing a fluidic access between the reservoir (1) and the reaction vessel (8), and
- a fluid line (12) extending at least in regions inside the reaction vessel (8) and which has an inlet line and an outlet line (14, 19), which are each fluid-tightly connectable or connected to a hose system (18) of the heat exchanger (W), and which, together with the hose system (18) of the heat exchanger (W), forms at least part of a fluid circuit;
**characterised in that** an expansion tank (3) having an overflow (6) into which the liquid from the reservoir (1) flows after fluidic access is generated between the reservoir (1) and the reaction vessel (8),
**in that** the overflow (6) provides a fluid connection (7) to the reaction vessel (8) through which a proportion of the stored liquid passes into the reaction vessel (8) to initiate an endothermal reaction with the reactant (11),
**in that** the fluid line (12) is fluidically connected to the expansion tank (3), into which a remaining proportion (26) of the stored liquid from the reservoir (1) passes, and
**in that** a fluid pump (16) is located along the fluid line (12), by means of which liquid from the expansion tank (3) passes into the fluid line (12) which extends at least in regions inside the reaction vessel (8).

2. The cooling unit according to claim 1,
**characterised in that** a non-return valve (10) is located along the fluid connection (7) of the overflow (6) and prevents a return flow of liquid from the reaction vessel (8) into the expansion tank (3).

3. The cooling unit according to claim 1 or 2,
**characterised in that** the fluid pump (16) is provided in the form of a suction unit located along the fluid line (12) outside the expansion tank and reaction vessel (3, 8).

4. The cooling unit according to claim 1 or 2,
**characterised in that** the fluid pump (16) is formed as a roller pump.

5. The cooling unit according to any one of claims 1 to 4,
**characterised in that** the fluid line (12) downstream of the region extending inside the reaction vessel (8) provides the outlet line (14), and
**in that** a filter unit (15) is located along the outlet line (14) extending outside the reaction vessel.

6. The cooling unit according to claim 5,
**characterised in that** the filter unit (15) is a bacteria filter.

7. The cooling unit according to any one of claims 1 to 6,
**characterised in that** the inlet line (19) of the fluid line (12) feeds into the expansion tank (3), and
**in that** the fluid circuit consists of the expansion tank (3), the fluid line (12) and the hose system of the heat exchanger (W), through which the remaining proportion (26) of the liquid circulates as heat transfer liquid of the heat exchanger (W).

8. The cooling unit according to any one of claims 1 to 7,
**characterised in that** the reservoir (1) is disposed vertically above the expansion tank (3), and
**in that** the functional means (4) is provided in the form of a sharp-edged object positioned vertically below the reservoir (1),
**in that** a spacer (25) is located between the reservoir (1) and the expansion tank (3) and vertically spaces apart the reservoir (1) above the functional means (4), and
**in that**, upon removal of the spacer (25), the reservoir (1) is driven by gravitational force to contact the functional means (4), which consequently mechanically penetrates the reservoir (1) locally, so that the liquid stored in the reservoir (1) pours fully into the expansion tank (3).

9. The cooling unit according to any one of claims 1 to 8,
**characterised in that** the functional means (4) is fixedly positioned on the expansion tank (3).

10. The cooling unit according to any one of claims 1 to 9,
**characterised in that** an agitator (20) is located inside the reaction vessel (8) and is driven via a mechanical interface by a drive motor (22) located outside the reaction vessel (8).

11. The cooling unit according to any one of claims 1 to 10,
**characterised in that** at least the reservoir (1), the functional means (4) and the reaction vessel (8) are disposable articles.

12. The cooling unit according to claims 5, 10 and 11,
**characterised in that** the filter unit (15) and the agitator (20) are parts of the disposable article and the drive motor (22) and the fluid pump (16) are connected to an electrical energy source (24) and an electrical controller (23) and are formed as a modular unit for a mechanical adaptation to the disposable article.

13. The cooling unit according to any one of claims 1 to 12,
**characterised in that** the heat exchanger (W) is integrated in a stationary or portable cooling system.

14. The cooling unit according to any one of claims 1 to 13,
**characterised in that** a binding agent is stored inside the reaction vessel (8) and, upon coming into contact with the liquid entering into the reaction vessel (8), can form a gel with the liquid.

15. The cooling unit according to any one of claims 1 to 14,
**characterised in that** at least the reservoir (1) and the reaction vessel (8) are made of a plastics-based lightweight packaging material.

16. The cooling unit according to claim 15,
**characterised in that** at least the inner wall of the reaction vessel which comes into contact with the liquid is provided with a liquid-tight coating.

17. The cooling unit according to any one of claims 1 to 16,
**characterised in that** the reaction vessel (8) provides a venting unit.

## Revendications

1. Unité de refroidissement, destinée à un échangeur thermique (W) intégré dans un oxygénateur (O), pour tempérer du sang conduit dans un circuit sanguin extracorporel, avec
- un réservoir de stockage (1) tenant en réserve un liquide,
- une cuve de réaction (8) comprenant un agent réactif (11), qui en association avec le liquide est susceptible d'initier une réaction endothermique,
- un moyen fonctionnel (4) créant un accès fluidique entre le réservoir de stockage (1) et la cuve de réaction (8), ainsi
- qu'une conduite à fluide (12) s'écoulant au moins par endroits à l'intérieur de la cuve de réaction (8), qui dispose d'une conduite d'alimentation et d'évacuation (14, 19) qui peut se relier ou est reliée de manière étanche au fluide avec un système de flexibles (18) de l'échangeur thermique (W) et qui avec le système de flexibles (18) de l'échangeur thermique (W) constitue au moins une partie d'un circuit de fluide,
**caractérisée en ce qu'**il est prévu un vase d'expansion (3) pourvu d'un trop-plein (6) dans lequel le liquide s'écoule à partir du réservoir de stockage (1), une fois que l'accès fluidique a été créé entre le réservoir de stockage (1) et la cuve de réaction (8),
**en ce que** le trop-plein (6) représente une liaison par fluide (7) vers la cuve de réaction (8), via laquelle une partie du liquide en réserve arrive dans la cuve de réaction (8), pour initier la réaction endothermique avec l'agent réactif (11),
**en ce que** la conduite à fluide (12) est fluidiquement reliée avec le vase d'expansion (3) dans lequel une partie résiduelle (26) du liquide en réserve arrive à partir du réservoir de stockage (1) et
**en ce que** le long de la conduite à fluide (12) est placée une pompe à fluide (16), par l'intermédiaire de laquelle du liquide arrive à partir du vase d'expansion (3) dans la conduite à fluide (12) s'écoulant au moins par endroits à l'intérieur de la cuve de réaction (8).

2. Unité de refroidissement selon la revendication 1,
**caractérisée en ce que** le long de la liaison par fluide (7) du trop-plein (6) est placé un clapet antiretour (10), qui empêche un reflux du liquide à partir de la cuve de réaction (8) dans le vase d'expansion (3).

3. Unité de refroidissement selon la revendication 1 ou 2,
**caractérisée en ce que** la pompe à fluide (16) est conçue sous la forme d'une unité aspirante, qui est placée la long de la conduite à fluide (12), à l'extérieur du vase d'expansion et de la cuve de réaction (3, 8).

4. Unité de refroidissement selon la revendication 1 ou 2,
**caractérisée en ce que** la pompe à fluide (16) est conçue sous la forme d'une pompe à rouleaux.

5. Unité de refroidissement selon l'une quelconque des revendications 1 à 4,
**caractérisée en ce que** la conduite à fluide (12) prévoit la conduite d'évacuation (14) en aval de la zone s'écoulant à l'intérieur de la cuve de réaction (8) et
**en ce que** le long de la conduite d'évacuation (14) s'écoulant à l'extérieur de la cuve de réaction est placée une unité filtrante (15).

6. Unité de refroidissement selon la revendication 5, **caractérisée en ce que** l'unité filtrante (15) est un filtre à bactéries, notamment un filtre à légionelles.

7. Unité de refroidissement selon l'une quelconque des revendications 1 à 6,
**caractérisée en ce que** la conduite d'alimentation (19) de la conduite à fluide (12) débouche dans le vase d'expansion (3) et
**en ce que** le circuit de fluide se compose du vase d'expansion (3), de la conduite à fluide (12) ainsi que du système de flexibles de l'échangeur thermique (W), à travers lequel la partie résiduelle (26) du liquide circule en tant que liquide caloporteur de l'échangeur thermique (W).

8. Unité de refroidissement selon l'une quelconque des revendications 1 à 7,
**caractérisée en ce que** le réservoir de stockage (1) est placé à la verticale au-dessus du vase d'expansion (3),
**en ce que** le moyen fonctionnel (4) est conçu sous la forme d'un objet à arêtes vives, qui est placé à la verticale en-dessous du réservoir de stockage (1),
**en ce qu'**entre le réservoir de stockage (1) et le vase d'expansion (3) est placée une entretoise (25), qui tient à distance le réservoir de stockage (1) à la verticale au-dessus du moyen fonctionnel (4) et
**en ce qu'**après le retrait de l'entretoise (25), sous l'effet de la force de gravité, le réservoir de stockage (1) entre en contact avec le moyen fonctionnel (4), qui par conséquent, traverse localement par effet mécanique le réservoir de stockage (1), de sorte que le liquide en réserve dans le réservoir de stockage (1) se déverse totalement dans le vase d'expansion (3).

9. Unité de refroidissement selon l'une quelconque des revendications 1 à 8,
**caractérisée en ce que** le moyen fonctionnel (4) est fixement placé sur le vase d'expansion (3).

10. Unité de refroidissement selon l'une quelconque des revendications 1 à 9,
**caractérisée en ce qu'**à l'intérieur de la cuve de réaction (8) est placé un agitateur (20), qui par l'intermédiaire d'une interface mécanique est susceptible d'être entraîné par un moteur d'entraînement (22) placé à l'extérieur de la cuve de réaction (8).

11. Unité de refroidissement selon l'une quelconque des revendications 1 à 10,
**caractérisée en ce qu'**au moins le réservoir de stockage (1), le moyen fonctionnel (4) et la cuve de réaction (8) sont conçus sous la forme d'un article jetable.

12. Unité de refroidissement selon les revendications 5, 10 et 11,
**caractérisée en ce que** l'unité filtrante (15) et l'agitateur (20) sont des composants de l'article jetable et le moteur d'entraînement (22) et la pompe à fluide (16) sont reliés avec une source d'énergie (24) électrique, ainsi qu'avec un système de commande (23) électrique et sont conçus sous la forme d'une unité modulaire pour une adaptation mécanique à l'article jetable.

13. Unité de refroidissement selon l'une quelconque des revendications 1 à 12,
**caractérisée en ce que** l'échangeur thermique (W) est intégré dans un groupe refroidisseur stationnaire ou portable.

14. Unité de refroidissement selon l'une quelconque des revendications 1 à 13,
**caractérisée en ce qu'**à l'intérieur de la cuve de réaction (8) est tenu en réserve un agent liant, qui lors d'un contact avec le liquide pénétrant dans la cuve de réaction (8) est susceptible de gélifier celui-ci.

15. Unité de refroidissement selon l'une quelconque des revendications 1 à 14,
**caractérisée en ce qu'**au moins le réservoir de stockage (1) et la cuve de réaction (8) sont fabriqués dans une matière d'emballage légère à base de matière plastique.

16. Unité de refroidissement selon la revendication 15,
**caractérisée en ce qu'**au moins la paroi intérieure de la cuve de réaction qui entre en contact avec le liquide est munie d'un revêtement étanche au liquide.

17. Unité de refroidissement selon l'une quelconque des revendications 1 à 16,
**caractérisée en ce que** la cuve de réaction (8) prévoit une unité de purge.
